# EUROPEAN PATENT APPLICATION

(11) **EP 4 744 711 A1**
(43) Date of publication of application: **20.05.2026**
(21) Application number: 25210256.1
(22) Date of filing: 22.10.2025
(51) Int. Cl.: A61M 39/12, A61M 39/10

(54) **TUBE CONNECTOR AND TUBE WITH CONNECTOR**

(30) Priority: 19.11.2024 JP 2024201799
(71) Applicant: Hirakawa Hewtech Corporation, Minato-ku Tokyo 108-0014 (JP)
(72) Inventor: SAKAMOTO, Sho, Koga-shi, 306-0232 (JP); TAKAE, Jun, Tokyo, 108-0014 (JP)
(74) Representative: Goddar, Heinz J.

(57) **Abstract**

A tube connector to connect a medical tube to a medical device includes a first member into which the medical tube is inserted, a second member that is screwed into the first member; and a seal ring that is housed in the first member, deforms with screwing of the second member into the first member, and fixes the medical tube by deformation, wherein, of the first member and the second member, at least the second member has light-transmission properties.

## Description

### CROSS-REFERENCE TO RELATED APPLICATIONS

The present application is based on Japanese patent application No. 2024-201799 filed on November 19, 2024, the entire contents of which are incorporated herein by reference.

### TECHNICAL FIELD

The present invention relates to a tube connector and a tube with connector.

### BACKGROUND OF THE INVENTION

There is a conventional tube connector that is composed of a first member (a compression member) and a second member (a main body) and is configured such that when the first member is screwed into the second member, a seal ring (a plug) is compressed and deformed by the two members and thereby fixed a medical tube (catheter) (see Patent Literature 1). This tube connector includes the first member and the second member respectively having an outer thread and an inner thread to be engaged with each other, and the seal ring attached to the first and second members, and is configured such that after a medical tube is inserted into a through-hole of the seal ring, the first member is screwed into the second member, which causes the seal ring to be compressed by the two members and the medical tube to be fixed with the seal ring deformed by the compression. This configuration of fixing the medical tube by deformation of the seal ring accompanying the screwing of the first member into the second member allows the medical tube to be easily fixed in a liquid-tight manner.

Citation List Patent Literature 1: JP S54-21087A

### SUMMARY OF THE INVENTION

However, the problem of the conventional tube connector is that when attaching the medical tube, it is not possible to know how far the tip of the medical tube has been inserted, hence, it is not possible to accurately position the tip of the medical tube. This causes problems in that the medical tube comes off the tube connector due to insufficient insertion of the medical tube, or the tip of the medical tube interferes with other components by inserting the medical tube too far.

Therefore, it is an object of the invention to provide a tube connector and a tube with connector that allow for accurate positioning of a tip of a medical tube at the time of attaching the medical tube.

To achieve the above object, the invention provides a tube connector to connect a medical tube to a medical device, the tube connector comprising:
a first member into which the medical tube is inserted;
a second member that is screwed into the first member; and
a seal ring that is housed in the first member, deforms with screwing of the second member into the first member, and fixes the medical tube by deformation,
wherein, of the first member and the second member, at least the second member has light-transmission properties.

To achieve the above object, the invention also provides a tube connector to connect a medical tube to a medical device, the tube connector comprising:
a first member into which the medical tube is inserted;
a second member that is screwed into the first member; and
a seal ring that is housed in the first member, deforms with screwing of the second member into the first member, and fixes the medical tube by deformation,
wherein the second member comprises a light-transmissive portion through which a position of a tip of the medical tube thereinside is visible.

To achieve the above object, the invention also provides a tube with connector, comprising:
a medical tube; and
a tube connector to connect the medical tube to a medical device,
wherein the tube connector comprises a first member into which the medical tube is inserted, a second member that is screwed into the first member, and a seal ring that is housed in the first member, deforms with screwing of the second member into the first member, and fixes the medical tube by deformation, and
wherein, of the first member and the second member, at least the second member has light-transmission properties.

To achieve the above object, the invention also provides a tube with connector, comprising:
a medical tube; and
a tube connector to connect the medical tube to a medical device,
wherein the tube connector comprises a first member into which the medical tube is inserted, a second member that is screwed into the first member, and a seal ring that is housed in the first member, deforms with screwing of the second member into the first member, and fixes the medical tube by deformation, and
wherein the second member comprises a light-transmissive portion through which a position of a tip of the medical tube thereinside is visible.

### Advantageous Effects of the Invention

The tube connector and the tube with connector of the invention allow for accurate positioning of a tip of a medical tube at the time of attaching the medical tube.

### BRIEF DESCRIPTION OF DRAWINGS

FIGS. 1A and 1B are diagrams illustrating a tube with connector in an embodiment of the present invention, wherein FIG. 1A is a front view when a medical tube is attached to a tube connector and FIG. 1B is a front view when the medical tube is detached from the tube connector.
FIG. 2A is a cross-sectional view showing the tube connector taken along line A-A'.
FIG. 2B is a cross-sectional view showing the tube connector taken along line B-B'.
FIG. 2C is an enlarged view of the main part of FIG. 2B, showing an area around an engagement raised portion and a retaining portion.
FIGS. 3A to 3C are diagrams illustrating a male member, wherein FIG. 3A is a front view, FIG. 3B is a right side view, and FIG. 3C is a left side view.
FIGS. 4A and 4B are diagrams illustrating behavior of an O-ring when pressed by a pressing portion, wherein FIG. 4A is an explanatory diagram as viewed from a side and FIG. 4B is a cross-sectional view taken along line C-C'.
FIGS. 5A to 5C are diagrams illustrating a female member, wherein FIG. 5A is a front view, FIG. 5B is a right side view, and FIG. 5C is a left side view.
FIGS. 6A to 6C are explanatory diagrams illustrating a tube attachment operation.
FIG. 7A is a front view showing the tube connector in the first modification.
FIG. 7B is a front view showing the tube connector in the second modification.

### DETAILED DESCRIPTION OF THE INVENTION

A tube connector and a tube with connector provided therewith in an embodiment of the invention will be described below with reference to the appended drawings. This tube with connector is composed of a medical tube used inside and outside human body, and a tube connector attached thereto. In particular, this tube with connector uses a tube connector configured so that the position of the tip of the medical tube thereinside is visible. Left and right, front and rear, and up and down in the following description are as defined in each drawing. In addition, in the present embodiment, the left-right direction coincides with an axial direction of the tube connector, and a direction orthogonal to the axial direction of the tube connector is the shear direction of the tube connector.

### Configuration of Tube with connector

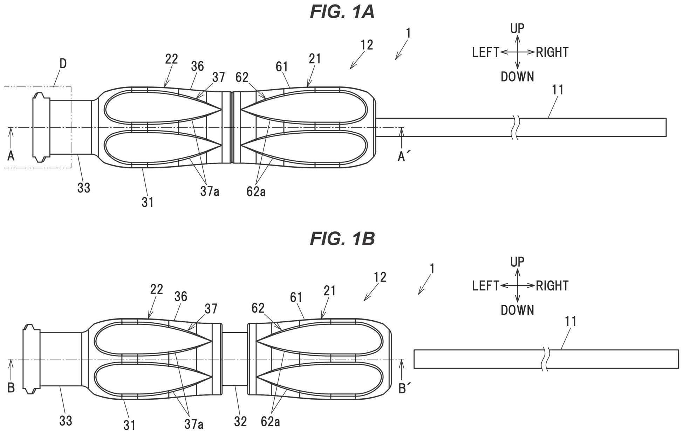

As shown in FIG. 1, a tube with connector 1 includes a medical tube 11, and a tube connector 12 that connects the medical tube 11 to a medical device D. An end of the medical tube 11 is inserted and fixed in the tube connector 12 which is in turn connected to the medical device D, and the medical tube 11 is thereby connected to the medical device D. The tube with connector 1 and the tube connector 12 described in the present embodiment are a tube with connector 1 for drainage and a tube connector 12 for drainage which are used in endoscopic biliary/pancreatic drainage procedures, as an example. Therefore, the medical device D to which the medical tube 11 is connected is assumed to be a syringe or a drainage bag (sterile bag), etc.

The medical tube 11 is a colored tube with colored exterior, and in the present embodiment, is assumed to be, e.g., a catheter that is introduced into the body of a patient. That is, in the present embodiment, one end of the medical tube 11 is introduced into the body of a patient, and the other end of the medical tube 11 is fixed to the tube connector 12 outside the body of the patient.

### Configuration of Tube connector

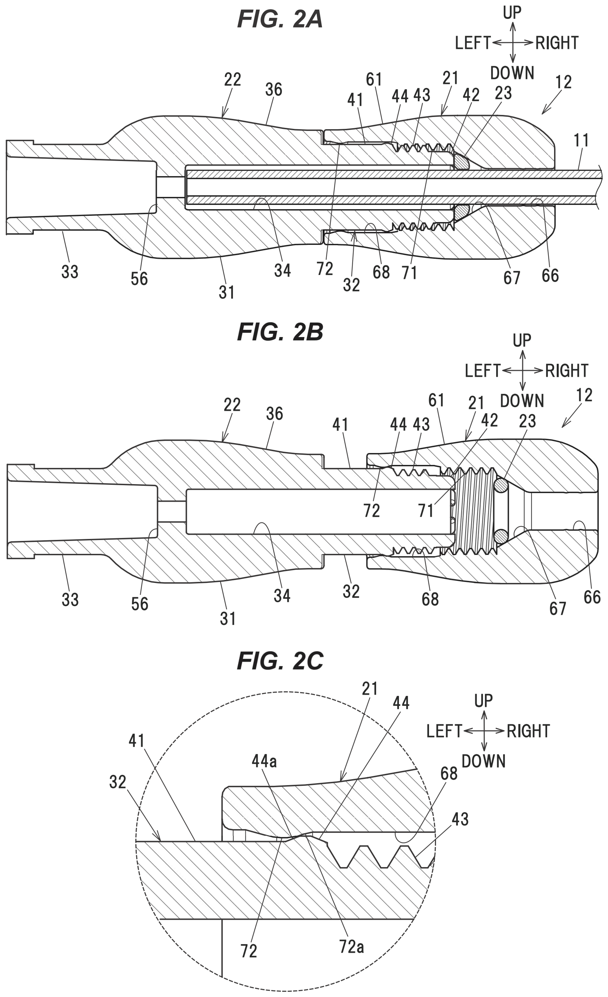

As shown in FIGS. 1 and 2, the tube connector 12 is formed in a bottle gourd shape as a whole, and includes a female member 21 on the right side into which the medical tube 11 is inserted, a male member 22 on the left side that is screwed into the female member 21, and an O-ring 23 that is housed in the female member 21, deforms with screwing of the male member 22 into the female member 21, and fixes (holds) the medical tube 11 by deformation. The female member 21 is an example of the first member, and the male member 22 is an example of the second member. Then, the O-ring 23 is an example of the seal ring that deforms with screwing of the male member 22 into the female member 21 and fixes the medical tube 11 by deformation. The O-ring 23 is a ring-shaped seal ring that is made of a synthetic rubber and has a circular cross-section.

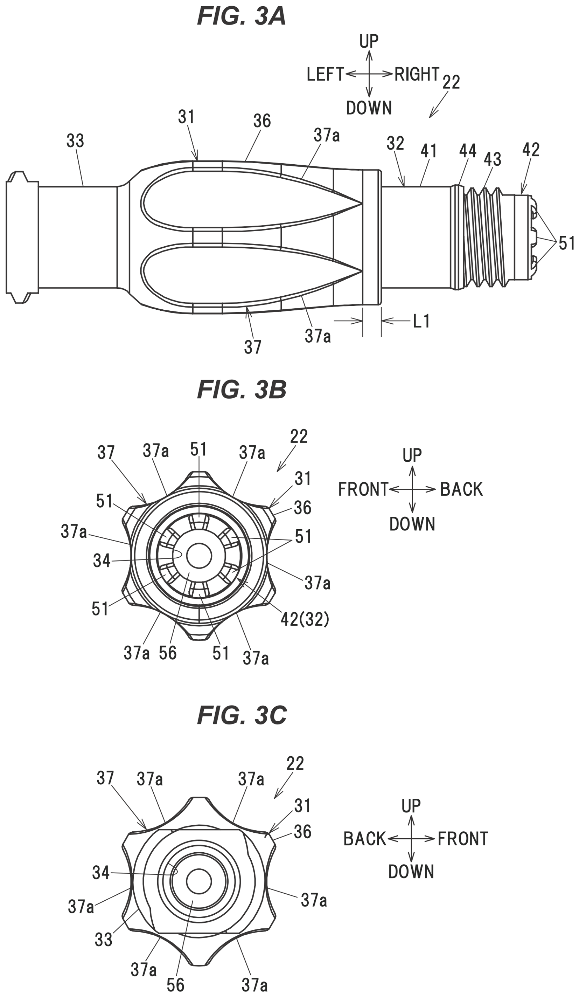

As shown in FIG. 3, the male member 22 has a male member main body 31, a shaft-shaped portion 32 protruding to the right from the male member main body 31, a device connection portion 33 protruding to the left from the male member main body 31 and connected to the medical device D, and a through-hole 34 penetrating the male member main body 31, the shaft-shaped portion 32 and the device connection portion 33. The male member main body 31, the shaft-shaped portion 32, the device connection portion 33 and the through-hole 34 are coaxially formed. The device connection portion 33 has a thread, etc. for connection and may be configured to be connected to the medical device D directly or through a separately-prepared medical tube or connector.

A screwing grip portion 37 for screwing the male member 22 into the female member 21 is formed on an outer circumferential surface 36 of the male member main body 31. The screwing grip portion 37 has a grip structure composed of six recessed portions 37a (finger placement portions) aligned in a circumferential direction, and is arranged at a position spaced leftward from a right end of the outer circumferential surface 36. A spacing distance L1 between the screwing grip portion 37 and the right end should be a distance at which the tips of gripping fingers when gripping the screwing grip portion 37 from the left side in a pinching manner do not stick out beyond the right end of the outer circumferential surface 36, and it is preferably, e.g., not less than 0.95 mm. Each of the recessed portions 37a is formed in, e.g., a drop shape with a pointy end on the right side.

The outer circumferential surface 36 of the male member main body 31 is tapered on the right end side and the outer shape of the right end is a true circle.

As shown in FIGS. 2 and 3, the shaft-shaped portion 32 has a shaft-shaped portion main body 41 having a cylindrical shape, a pressing portion 42 that is formed at the right end of the shaft-shaped portion main body 41 and presses the O-ring 23 with screwing of the male member 22 into the female member 21, a male-side thread portion 43 formed on the outer circumferential surface of the shaft-shaped portion main body 41 so as to be adjacent to the pressing portion 42 on the left side, and an engagement raised portion 44 formed on the outer circumferential surface of the shaft-shaped portion main body 41 so as to be adjacent to the male-side thread portion 43 on the left side. The male-side thread portion 43 threadedly engages a female-side thread portion 71 (described later) of the female member 21.

As shown in FIG. 2C, the engagement raised portion 44 is formed so as to protrude in a radial direction over the entire circumference of the shaft-shaped portion main body 41, and has a trapezoidal cross-sectional shape. The engagement raised portion 44 and a retaining portion 72 (described later) of the female member 21 constitute a separation suppression structure. The details thereof will be described later.

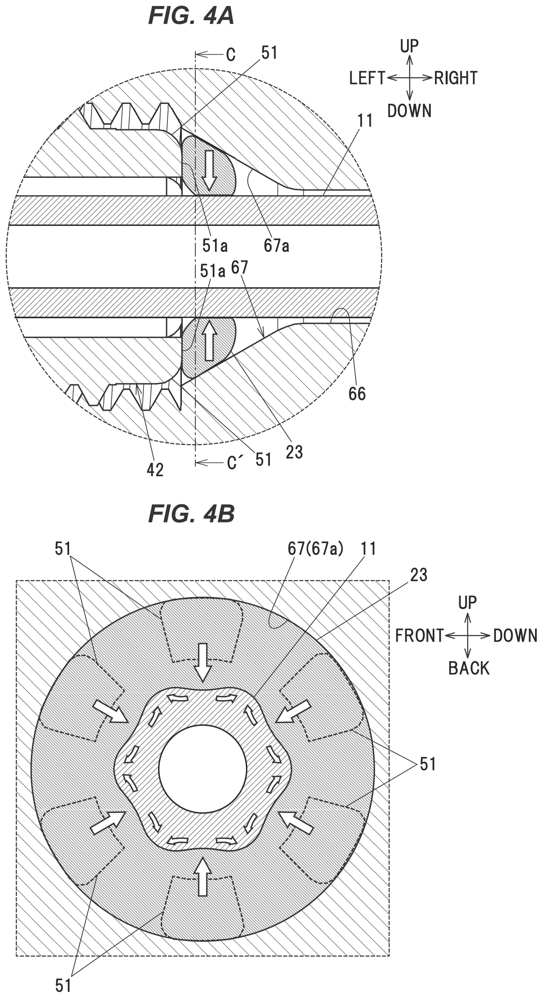

As shown in FIGS. 3 and 4, the pressing portion 42 has six pressing pieces 51 arranged with predetermined spaces in the circumferential direction, and the six pressing pieces 51 press the O-ring 23 through which the medical tube 11 is inserted. In detail, the O-ring 23 is pressed by the six pressing pieces 51 at six locations spaced in the circumferential direction. It is thereby possible to greatly deform the O-ring 23 at the six locations, and the hole of the O-ring 23 becomes substantially hexagonal in shape, as shown in FIG. 4B. As a result, the medical tube 11 inserted through the hole of the O-ring 23 is pressed by the O-ring 23 at only six locations corresponding to the pressing pieces 51, which causes the wall of a tube main body (e.g., an outer layer tube) of the medical tube 11 to escape to non-pressed areas (areas corresponding to the spaces) and the tube main body to become substantially hexagonal in shape. It is thereby possible to suppress blocking of the medical tube 11 and also firmly fix the medical tube 11. It is preferable that each of the spaces between the six pressing pieces 51 have a circumferential width similar to (substantially the same as) a circumferential width of each pressing piece 51. For example, it is preferable that each of the spaces between the six pressing pieces 51 have a circumferential width that is not less than 0.8 times and not more than 1.2 times the circumferential width of each pressing piece 51.

In addition, as shown in FIG. 4A, each pressing piece 51 of the pressing portion 42 has a pressing surface 51a formed parallel to a shear direction of the tube connector 12. That is, the pressing surfaces 51a of the pressing portion 42 are surfaces substantially perpendicular to the axial direction of the tube connector 12.

As shown in FIGS. 2 and 3, the through-hole 34 serves as an insertion hole for insertion of the medical tube 11, and also serves as a communication flow path through which an internal flow path of the medical tube 11 communicates with the medical device D. That is, when the medical tube 11 is inserted into and fixed in the tube connector 12, the internal flow path of the medical tube 11 communicates with the medical device D through the through-hole 34 of the tube connector 12 in a liquid-tight manner, and the medical tube 11 is connected to the medical device D.

In addition, at the middle position in the axial direction, the through-hole 34 has a tube restriction portion 56 which is formed as a narrowed portion of the through-hole 34 and against which a tip of the medical tube 11 inserted into the female member 21 butts. The tube restriction portion 56 is an annular rib that protrudes from an inner circumferential surface of the through-hole 34, and an annular-shaped right surface of the tube restriction portion 56 serves as a butting surface against which the medical tube 11 butts. An inner diameter of the tube restriction portion 56 is smaller than an outer diameter of the medical tube 11 and larger than an inner diameter of the medical tube 11. Thus, the tube restriction portion 56 serves as a stopper to restrict the tip of the medical tube 11 from being inserted beyond a predetermined insertion position, and also serves as a positioning portion against which the tip of the medical tube 11 butts when the medical tube 11 is inserted into the tube connector 12 so that the tip of the medical tube 11 is positioned. In this manner, by providing the tube restriction portion 56, it is possible to easily position the tip of the medical tube 11 inserted into the tube connector 12.

The male member 22 (the male member main body 31, the shaft-shaped portion 32 and the device connection portion 33) is made of a translucent resin material and has light-transmission properties. Thus, the male member 22 is configured so that the tip of the medical tube 11 thereinside is visible. This makes it possible to more easily and accurately position the tip of the medical tube 11 inserted into the tube connector 12.

### Configuration of Female member

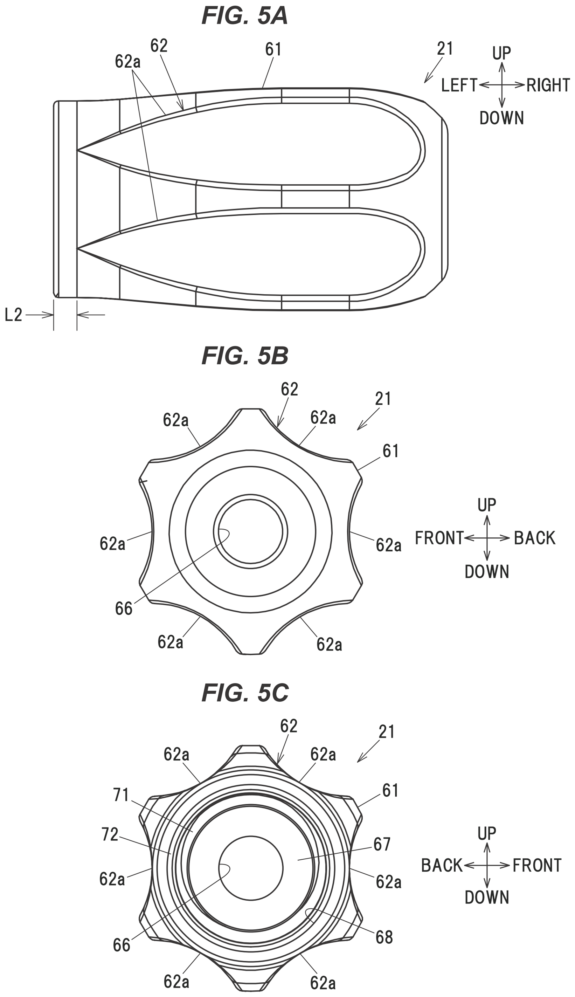

As shown in FIG. 5, the female member 21 is formed in a tapered cylindrical shape, and a screwing grip portion 62 for screwing the male member 22 into the female member 21 is formed on an outer circumferential surface 61 of the female member 21. The screwing grip portion 62 has a grip structure composed of six recessed portions 62a (finger placement portions) aligned in the circumferential direction, and is arranged at a position spaced rightward from a left end of the outer circumferential surface 61. A spacing distance L2 between the screwing grip portion 62 and the left end should be a distance at which the tips of gripping fingers when gripping the screwing grip portion 62 from the right side in a pinching manner do not stick out beyond the left end of the outer circumferential surface 61, and it is preferably, e.g., not less than 0.95 mm. Each of the recessed portions 62a is formed in, e.g., a drop shape with a pointy end on the left side. When screwing the male member 22 into the female member 21, the screwing grip portion 62 of the female member 21 is held in a pinching manner by one hand with fingers placed on the recessed portions 62a, the screwing grip portion 37 of the male member 22 is held in a pinching manner by the other hand with fingers placed on the recessed portions 37a, and the male member 22 is rotated relative to the female member 21, thereby screwing the male member 22 into the female member 21.

The outer circumferential surface 61 of the female member 21 is tapered on the left end side and the outer shape of the left end is a true circle having the same diameter as the outer shape of the right end of the male member main body 31. The shape of the left end portion of the outer circumferential surface 61 of the female member 21 and the shape of the right end portion of the outer circumferential surface 36 of the male member 22 form a recess at the left-right center of the entire tube connector 12, and this recess serves as a connector gripping portion to grip the tube connector 12 as one unit.

Then, as shown in FIGS. 2 and 5, inside the female member 21, an insertion hole portion 66 for insertion of the medical tube 11, a tapered hole-shaped seating portion 67 connected to the left side of the insertion hole portion 66 to seat the O-ring 23, and a receiving hole portion 68 connected to the left side of the seating portion 67 to receive the shaft-shaped portion 32 of the male member 22 are coaxially formed from the right side.

The female-side thread portion 71 to be threadedly engaged with the male-side thread portion 43 of the male member 22, and the retaining portion 72 arranged on the left side of the female-side thread portion 71 at a distance therefrom to mechanically stop the engagement raised portion 44 of the male member 22, are formed on the inner circumferential surface of the receiving hole portion 68. The male member 22 is screwed into the female member 21 by threadedly engaging the male-side thread portion 43 of the male member 22 with the female-side thread portion 71 of the female member 21.

As shown in FIG. 2C, the retaining portion 72 is formed so as to protrude in the radial direction over the entire circumference of the receiving hole portion 68, and has a trapezoidal cross-sectional shape. A right surface 72a of the retaining portion 72 serves as a stop surface that engages a left surface 44a of the engagement raised portion 44 to mechanically stop the male member 22 in a direction of separation of the male member 22 from the female member 21 (in the leftward direction), and the right surface 72a comes into contact with the left surface 44a of the engagement raised portion 44 and mechanically stops the engagement raised portion 44 in the direction of separation, thereby restricting the male member 22 from separating from the female member 21. In this way, since portions of joining the female member 21 and the male member 22 are provided in addition to the thread portions 43 and 71, separation of the female member 21 from the male member 22 can be suppressed even if loosening the threaded engagement between the thread portions 43 and 71 causes release of the threaded engagement between the thread portions 43 and 71, hence, unintentional separation of the female member 21 from the male member 22 can be suppressed. In this regard, a spacing distance between the female-side thread portion 71 and the retaining portion 72 is preferably not less than the length of the male-side thread portion 43 so that separation of the female member 21 from the male member 22 can be suppressed even if the threaded engagement between the thread portions 43 and 71 is released.

The right surface 72a of the retaining portion 72 is an inclined surface that is radially inwardly sloped up in the leftward direction (in the direction of separation). Therefore, the male member 22 can be intentionally separated from the female member 21 by pulling the male member 22 hard in the direction of separation relative to the female member 21.

As shown in FIG. 2, the seating portion 67 has a seating surface 67a formed in an inclined shape (a tapered hole shape) that is radially outwardly sloped down toward the left, and the O-ring 23 is seated on the seating surface 67a. That is, the seating surface 67a of the seating portion 67 is formed in an inclined shape leaning from a plane parallel to the pressing surfaces 51a of the pressing portion 42 toward the medical tube 11. As shown in FIG. 4A, the pressing surfaces 51a of the pressing portion 42 are formed parallel to the shear direction, and the seating surface 67a for the O-ring 23 is an inclined surface leaning toward the medical tube 11. Therefore, when the O-ring 23 seated on the seating surface 67a is pressed by the pressing portion 42, stress toward the medical tube 11 is generated in the O-ring 23 which thus greatly deforms toward the medical tube 11. The medical tube 11 can thereby be firmly fixed.

The female member 21 is also made of a translucent resin material and has light-transmission properties. Thus, the female member 21 is configured so that the medical tube 11 and the O-ring 23 thereinside are visible.

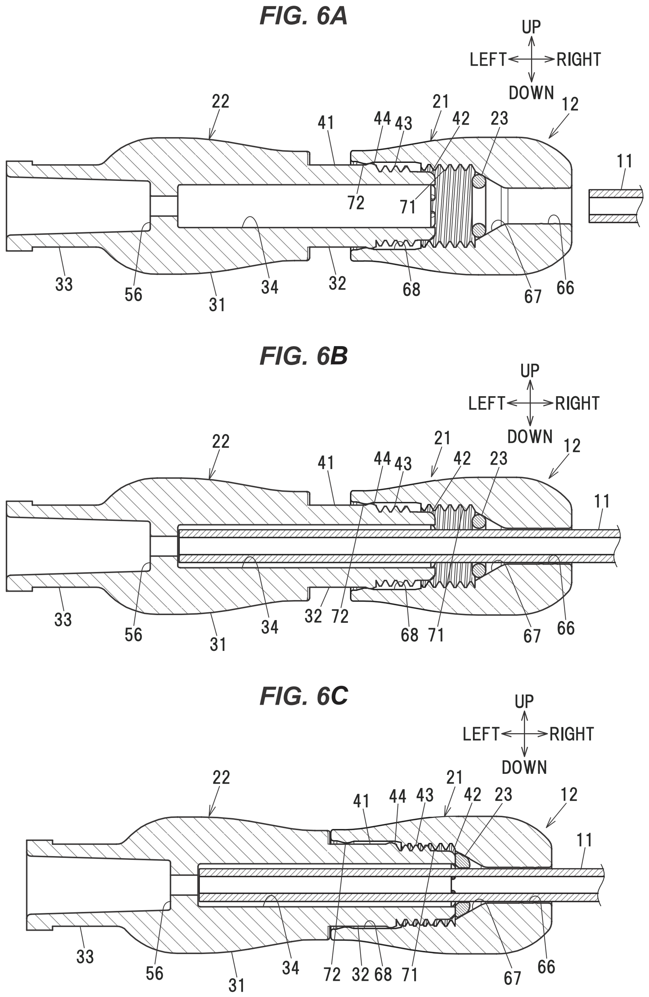

Next, a tube attachment operation of attaching the medical tube 11 to the tube connector 12 will be described with reference to FIGS. 6A to 6C. As shown in FIG. 6A, this tube attachment operation is performed in a state in which the female member 21 and the male member 22 are combined by the retaining portion 72, i.e., in a state in which the shaft-shaped portion 32 of the male member 22 is received in the receiving hole portion 68 of the female member 21 and the engagement raised portion 44 is located to the right of the retaining portion 72.

In the tube attachment operation, first, the tip of the medical tube 11 is inserted into the female member 21 and the male member 22 through the insertion hole portion 66 of the female member 21, and the medical tube 11 is thereby inserted through the hole of the O-ring 23, as shown in FIG. 6B. At this time, the tip of the medical tube 11 butts against the tube restriction portion 56, and the tip of the medical tube 11 is thereby positioned. The tip of the medical tube 11 can also be positioned while visually checking since the female member 21 and the male member 22 have light-transmission properties.

Once the medical tube 11 is inserted, the medical tube 11 is fixed with the O-ring 23 by screwing the male member 22 into the female member 21, as shown in FIG. 6C. That is, when the male member 22 is screwed into the female member 21 through the male-side thread portion 43 and the female-side thread portion 71, the pressing portion 42 presses the O-ring 23 which in turn deforms inward due to this pressure and fixes the medical tube 11 inserted through the O-ring 23. Since the pressing surfaces 51a of the pressing portion 42 (the six pressing pieces 51) are parallel to the shear direction while the seating surface 67a of the seating portion 67 is formed at an angle, the radially inward deformation of the O-ring 23 is promoted at this time as shown in FIG. 4A and the medical tube 11 is firmly fixed. In addition, by pressing the O-ring 23 with the six pressing pieces 51 arranged with spaces in the circumferential direction, the O-ring 23 greatly deforms radially inward only at the positions of the pressing pieces 51, as shown in FIG. 4B. This causes the O-ring 23 to press the outer circumferential surface of the medical tube 11 only at positions corresponding to the pressing pieces 51, allowing the wall of the medical tube 11 to escape to non-pressed areas (areas corresponding to the spaces) and blocking of the medical tube 11 to be avoided. The tube attachment operation then ends.

### Functions and Effects of the embodiment

In the configuration of the above embodiment, the male member 22 has light-transmission properties, hence, the medical tube 11 inside the male member 22 is visible. Thus, when attaching the medical tube, the tip of the medical tube 11 can be positioned while visually checking the position of the tip of the medical tube 11. It is thereby possible to accurately position the tip of the medical tube when attaching the medical tube.

In addition, since the female member 21 has light-transmission properties, the medical tube 11 and the O-ring 23 inside the female member 21 are visible. It is thereby possible to check the position and state of the medical tube 11 and the O-ring 23 inside the female member 21.

In addition, since the female member 21 and the male member 22 have light-transmission properties, it is possible to easily check whether or not the medical tube 11 is inserted through the O-ring 23.

In addition, by providing the tube restriction portion 56, it is possible to suppress interference of the tip of the medical tube 11 with the medical device D caused by inserting the medical tube 11 too far.

In addition, since the inner diameter of the tube restriction portion 56 is smaller than the outer diameter of the medical tube 11 and larger than the inner diameter of the medical tube 11 (the tube main body), narrowing of the flow path at the tube restriction portion 56 can be avoided while maintaining the restriction function of the tube restriction portion 56.

### Other embodiments

Although the embodiment of the invention has been described, the invention according to claims is not to be limited to the embodiment. In addition, not all combinations of the features described in the embodiment are necessary to solve the problem of the invention. The invention can be appropriately modified and implemented without departing from the gist thereof.

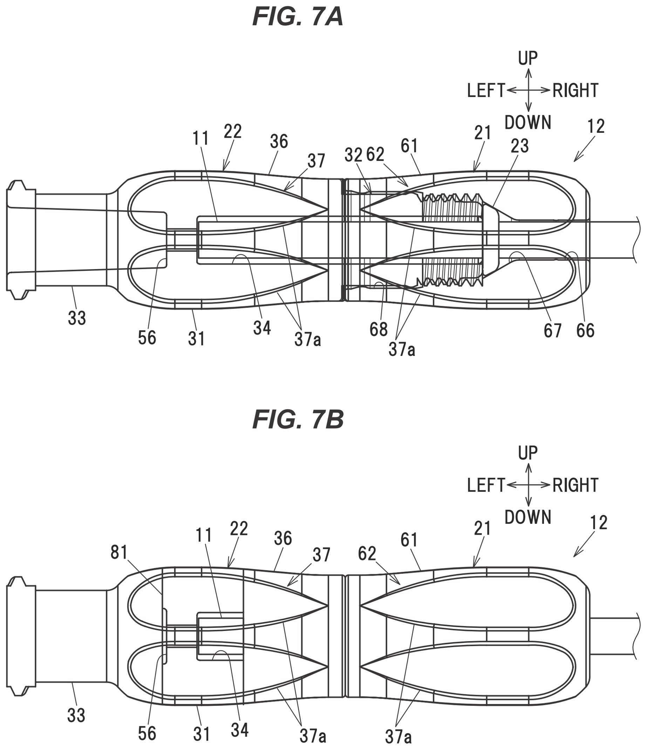

For example, the female member 21 and the male member 22 are translucent in the above embodiment, but the configuration is not limited thereto as long as the female member 21 and the male member 22 have light-transmission properties. That is, the configuration may be such that the female member 21 and the male member 22 are transparent, as shown in FIG. 7A.

In addition, the configuration in the above embodiment is such that both the female member 21 and the male member 22 have light-transmission properties, but the configuration may be such that only one of the female member 21 and the male member 22 has light-transmission properties.

In addition, the configuration in the above embodiment is such that the entire male member 22 has light-transmission properties, but the configuration may be such that a portion of the male member 22 has light-transmission properties. That is, the male member 22 may have a light-transmissive portion 81 that is a transparent portion or a translucent portion through which the position of the tip of the medical tube 11 thereinside is visible, as shown in FIG. 7B. In such a case, the light-transmissive portion 81 is preferably arranged in the circumferential direction around the tube restriction portion 56, as shown in FIG. 7B.

In addition, in the above embodiment, the female member 21 is provided on the side to which the medical tube 11 is connected, and the male member 22 is provided on the side to which the medical device D is connected. However, the configuration may be such that the female member 21 is provided as a member on the side to which the medical device D is connected, and the male member 22 is provided on the side to which the medical tube 11 is connected.

In addition, the tube with connector 1 and the tube connector 12 which are used for the purpose of drainage in endoscopic biliary/pancreatic drainage procedures have been described as examples in the above embodiment, but the invention is not limited thereto. That is, the invention may be applied to a tube with connector 1 and a tube connector 12 which are used for purposes other than drainage.

In addition, a catheter that is introduced into the body of a patient has been described as an example of the medical tube 11 in the above embodiment, but the invention may be applied to a medical tube 11 used outside the body. That is, the invention may be applied to a tube with connector 1 having a medical tube 11 used outside the body, or a tube connector 12 for a medical tube 11 used outside the body.

## Claims

1. A tube connector to connect a medical tube to a medical device, the tube connector comprising:
a first member into which the medical tube is inserted;
a second member that is screwed into the first member; and
a seal ring that is housed in the first member, deforms with screwing of the second member into the first member, and fixes the medical tube by deformation,
wherein, of the first member and the second member, at least the second member has light-transmission properties.

2. The tube connector according to claim 1, wherein the second member comprises a tube restriction portion against which a tip of the medical tube inserted into the first member butts.

3. The tube connector according to claim 1, wherein the first member and the second member have light-transmission properties.

4. The tube connector according to claim 2, wherein the first member and the second member have light-transmission properties.

5. A tube connector to connect a medical tube to a medical device, the tube connector comprising:
a first member into which the medical tube is inserted;
a second member that is screwed into the first member; and
a seal ring that is housed in the first member, deforms with screwing of the second member into the first member, and fixes the medical tube by deformation,
wherein the second member comprises a light-transmissive portion through which a position of a tip of the medical tube thereinside is visible.

6. A tube with connector, comprising:
a medical tube; and
a tube connector to connect the medical tube to a medical device,
wherein the tube connector comprises a first member into which the medical tube is inserted,
a second member that is screwed into the first member, and a seal ring that is housed in the first member, deforms with screwing of the second member into the first member, and fixes the medical tube by deformation, and
wherein, of the first member and the second member, at least the second member has light-transmission properties.

7. A tube with connector, comprising:
a medical tube; and
a tube connector to connect the medical tube to a medical device,
wherein the tube connector comprises a first member into which the medical tube is inserted,
a second member that is screwed into the first member, and a seal ring that is housed in the first member, deforms with screwing of the second member into the first member, and fixes the medical tube by deformation, and
wherein the second member comprises a light-transmissive portion through which a position of a tip of the medical tube thereinside is visible.
